# EUROPÄISCHE PATENTSCHRIFT

(11) **EP 3 554 615 B1**
(45) Veröffentlichungstag und Bekanntmachung des Hinweises auf die Patenterteilung: **20.12.2023**
(21) Anmeldenummer: 17794703.3
(22) Anmeldetag: 26.10.2017
(51) Int. Cl.: A61M 35/00, A61H 33/14, A61N 1/44

(54) **VORRICHTUNG ZUR HUMAN- UND TIERMEDIZINISCHEN BEHANDLUNG UND APPLIKATIONSEINRICHTUNG DAFÜR**
DEVICE FOR THE MEDICAL TREATMENT OF HUMANS AND ANIMALS, AND APPLICATION DEVICE FOR SAME
SYSTÈME POUR LE TRAITEMENT MÉDICAL D'UN ÊTRE HUMAIN ET D'UN ANIMAL ET DISPOSITIF D'APPLICATION POUR CE SYSTÈME

(30) Priorität: 14.12.2016 DE 102016014942
(43) Veröffentlichungstag der Anmeldung: 23.10.2019
(73) Patentinhaber: Lohmann&Rauscher GmbH, 1140 Wien (AT); Relyon Plasma GmbH, 93055 Regensburg (DE)
(72) Erfinder: BUCHEGGER, Patricia, 2801 Katzelsdorf (AT); BURGER, Dominik, 93087 Alteglofsheim (DE); KORZEC, Dariusz, 93167 Falkenstein (DE); NETTESHEIM, Stefan, 93047 Regensburg (DE); ROHRER, Christian, 9500 Villach (AT); HOPPENTHALER, Florian, 94365 Parkstetten (DE); MERZ, Katharina, 85375 Neufahrn (DE)
(74) Vertreter: Seranski, Klaus
(86) Internationale Anmeldenummer: PCT/EP2017/077499
(87) Internationale Veröffentlichungsnummer: WO 2018/108373

(56) Entgegenhaltungen:
- WO-A1-98/10825
- DE-A1-102011 001 416
- US-A1- 2016 296 738

## Beschreibung

Die Erfindung betrifft eine Vorrichtung zur human- und/oder tiermedizinischen Behandlung, insbesondere zur Behandlung von Wunden und/oder Dermatosen, mit einem zum Erzeugen reaktiver Gase ausgelegten Reaktionsgasgenerator und einer Applikationseinrichtung zum Applizieren der reaktiven Gase in einen Behandlungsbereich sowie eine Applikationseinrichtung für eine derartige Vorrichtung.

Bei der therapeutischen Behandlung kommen neben herkömmlichen pharmazeutischen Produkten und physikalischen Behandlungsansätzen, wie etwa der Kompressions- und/oder Unterdrucktherapie, in der jüngeren Vergangenheit auch Behandlungsansätze zum Einsatz, bei denen reaktive Gase eingesetzt werden. Entsprechende Behandlungsansätze sind auf dem Gebiet der Dermatologie zur Behandlung von Dermatosen und Wunden jeglicher Art, von onkologischen Erkrankungen sowie in der Zahnmedizin bekannt geworden. Im Zuge der Plasmabehandlung werden reaktive Gasspezies gebildet und gezielt über dem Behandlungsbereich angereichert. Das stellt einen möglichen physikalischen Behandlungsansatz dar. Reaktive Gase enthalten stark reaktive Komponenten. Diese entfalten ihr oxidatives Potential an der Oberfläche, wo sie unselektiv Proteine, Lipide und Nukleinsäuren oxidieren. Höhere eukaryotische Zellen verfügen über hochentwickelte Abwehrmechanismen und können mit dem verursachten oxidativen Stress wesentlich besser umgehen als Bakterien, Pilze oder Viren. Zu diesen Abwehrmechanismen zählen unter anderem Antioxidantien, ROS (Reactive Oxygen Species) abbauende Enzyme, wie Katalasen oder Dismutasen sowie die DNA-Reparaturmaschinerie,. Daher sind reaktive Gase insbesondere bei der Behandlung von Wunden und Dermatosen mit Erfolg einsetzbar.

Während der Plasmabehandlung werden die reaktiven Gase üblicherweise durch die Übertragung von ausreichend Energie in einer Gasentladung gebildet. In einer solchen Entladung entsteht ein Plasma aus teilweise oder vollständig geladenen Teilchen, aus denen reaktive Gasspezies hervorgehen.. Plasma wird häufig in elektrostatischen oder elektromagnetischen Feldern, z.B. durch Wechsel- oder Gleichstromanregung oder Mikrowellenanregung, erzeugt. Für regenerative medizinische Zwecke werden üblicherweise kalte Atmosphärendruckplasmen eingesetzt. Sofern Luft als Reaktionsgas eingesetzt wird, entstehen als Produkte des Plasmas überwiegend reaktive Sauerstoff- und Stickstoffspezies wie zum Beispiel Ozon (O₃) und Wasserstoffperoxid (H₂O₂) oder Stickoxide mit stark oxidierender Wirkung. Daneben entstehen im Plasma selbst auch Elektronen und Ionen, sowie bei der Relaxation der angeregten Plasmabestandteile emittierte Photonen, welche selbst eine antiseptische und wundheilungsfördernde Wirkung entfalten können. Kaltes Atmosphärendruckplasma kann durch dielektrisch behinderte Entladung zwischen isolierend umhüllten Elektroden, durch Mikrowellenanregung oder einen piezoelektrischer Transformator als aktives Dielektrikum in einem gasgefüllten Raum gebildet werden. Bei der Plasmatherapie unterscheidet man grundsätzlich zwischen den folgenden Behandlungsansätzen:

### 1. Die direkte Plasmatherapie

Bei diesem Therapieansatz wird das Plasma im Behandlungs- bzw. Wundbereich selbst üblicherweise im Wege einer dielektrisch behinderten Entladung erzeugt, wobei der Behandlungsbereich bzw. die Haut als Elektrode bzw. Gegenelektrode bei der Plasmaerzeugung mittels Gasentladung eingesetzt werden kann. Vorrichtungen zur direkten Plasmabehandlung sind beispielsweise in der WO2011/023478, der DE 10 2011 001 416 A1 und der WO2015/184395 beschrieben.

### 3. Die indirekte Plasmatherapie

Bei der indirekten Plasmatherapie wird das Plasma in einem separaten Plasmagenerator erzeugt und via Diffusion oder ggf. mittels Druckluft, Pumpe oder Ventilator zum Behandlungsbereich geführt. Dabei können die in dem Plasma erzeugten reaktiven Gase auch über Leitungselemente, wie etwa Schläuche oder dergleichen, zum Behandlungsbereich geführt werden. Entsprechende Vorrichtungen für die indirekte Plasmatherapie sind in der DE 10 2012 003 563 A1 beschrieben.

Bei der in der DE 10 2012 003 563 A1 beschriebenen Vorrichtung werden die reaktiven Gase in einem in einem Gehäuse angeordneten Plasmagenerator erzeugt. Mit Hilfe eines in dem Gehäuse angeordneten Strömungsmoduls werden die reaktiven Gase in Form eines Freistrahls zum Behandlungsbereich gefördert. Zur planmäßigen Beeinflussung des Freistrahls ist gemäß der genannten Schrift eine Strahlsteuereinheit mit einem darüber steuerbaren Leitapparat vorgesehen. Dadurch soll erreicht werden, dass der das desinfizierende Plasma transportierende Gasstrom so eingestellt wird, dass das Plasma nur in einem lokal begrenzten Bereich, nämlich dem zu behandelnden Wundfeld, wirksam ist. Dadurch soll das OP-Personal geschützt werden, dass bei einer weiträumigen Verteilung des Plasmas einer nicht tolerierbaren Dauerbelastung ausgesetzt sein würde.

In diesem Zusammenhang kann darauf hingewiesen werden, dass die Geruchsschwelle für in dem Plasma gebildetes Ozon extrem niedrig ist und bei ca. 15 ppb liegt. Der Ozongeruch wird vielfach als sehr störend und unangenehm wahrgenommen. Eine dauerhaft erhöhte (viel größer als 50 ppb) Ozonbelastung kann zu Hustenreiz, Atemwegsproblemen und Kopfschmerzen führen. In der WO89/10825 ist die Verwendung von Ozon für die Herstellung gasförmiger Medikamente beschrieben, wobei eine Filtereinrichtung zum Filtern des vom Behandlungsgebiet abgezogenen Gases vorgesehen sein kann.

Wenngleich mit den in der genannten Schrift beschriebenen offenen Systemen zur indirekten Plasmabehandlung ein Schutz für das OP-Personal erreicht werden kann, hat es sich gezeigt, dass die Belastung bei der Plasmabehandlung immer noch kaum akzeptabel ist.

Angesichts dieser Probleme im Stand der Technik liegt der Erfindung die Aufgabe zugrunde, eine Vorrichtung zur human- und/oder tiermedizinischen Behandlung unter Verwendung reaktiver Gase bereitzustellen, mit der unter Gewährleistung einer zufriedenstellenden Behandlung die Belastung von Ärzten, Schwestern und Pflegern mit reaktiven Gasen, wie etwa Ozon, reduziert werden kann.

Erfindungsgemäß wird diese Aufgabe durch die in Patentanspruch 1 angegebene Weiterbildung von Vorrichtungen für die indirekte Plasmatherapie gelöst. Vorteilhafte Weiterbildungen der Erfindung sind in den abhängigen Patentansprüchen angegeben.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. in der Zeichnung zeigt:
- Fig. 1: eine Behandlungsvorrichtung zur Plasmatherapie,
- Fig. 2: eine Behandlungsvorrichtung gemäß einer ersten Ausführungsform der Erfindung,
- Fig. 3: eine Behandlungsvorrichtung gemäß einer zweiten Ausführungsform der Erfindung,
- Fig. 4: eine weitere Behandlungsvorrichtung gemäß einer dritten Ausführungsform der Erfindung,
- Fig. 5: eine Behandlungsvorrichtung gemäß einer vierten Ausführungsform der Erfindung,
- Fig. 6: eine Behandlungsvorrichtung gemäß einer fünften Ausführungsform der Erfindung, und
- Fig. 7: den Wirkungsgrad der Neutralisierungseinrichtung in einer Ausführungsform.

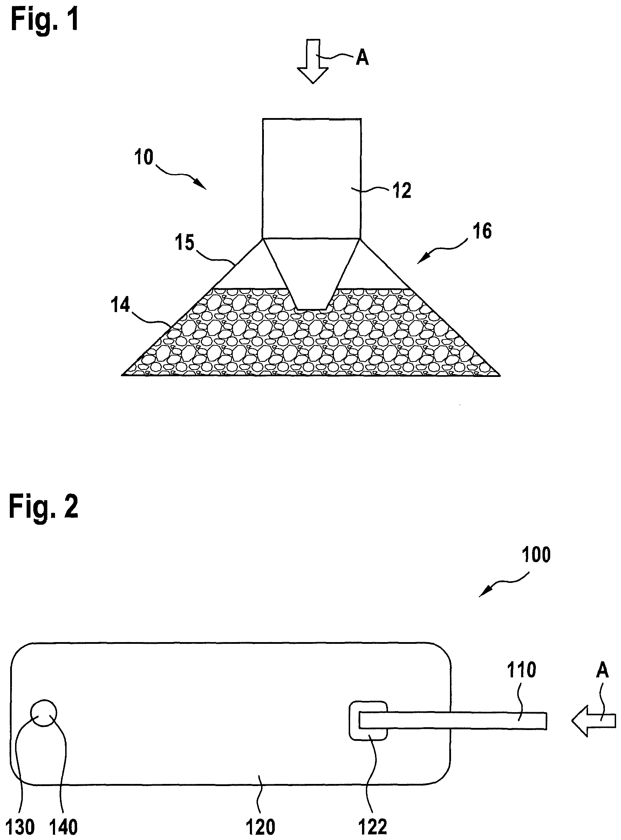

Die in Fig. 1 dargestellte Behandlungsvorrichtung umfasst eine Düseneinrichtung 12, der reaktive Gase aus einem in Form einer Plasmaquelle ausgeführten Reaktionsgasgenerator zugeführt werden, wie durch den Pfeil A angedeutet. Mit der Düseneinrichtung 12 werden die reaktiven Gase in Form eines offenen Gasstrahls zur Wundoberfläche gefördert. Auf die Düseneinrichtung 12 ist eine trichterförmige Gasführungsvorrichtung aufgesteckt. Die Düseneinrichtung 12 und Gasführungsvorrichtung 16 bilden zusammen eine Applikationseinrichtung im Sinne der Erfindung.

Die Gasführungseinrichtung umfasst einen sich in Richtung auf die zu behandelnde Wunde erweiternden Kunststofftrichter 15, der an seiner äußeren Begrenzungsfläche von einem mit Aktivkohle versehenen offenporigen Schaum 14 abgedeckt ist, wobei sich der offenporige Schlauch 14 über den Rand des Kunststofftrichters 15 hinaus in Richtung auf die Wunde erstreckt. Mit Hilfe des mit Aktivkohle versetzten offenporigen Schaums werden die den Behandlungsbereich verlassenden reaktiven Gase neutralisiert, d. h. zu stabilen Stoffen umgesetzt. Beispielsweise wird Ozon mit Aktivkohle zu Sauerstoff und Kohlendioxid umgesetzt.

Bei der in Fig. 2 dargestellten Ausführungsform der Erfindung werden die in einem Reaktionsgasgenerator in Form einer Plasmaquelle hergestellten reaktiven Gase, wie durch den Pfeil A angedeutet, über einen Schlauch 110 in einen Folienschlauchbeutel 120 eingeleitet. Dabei ist auf den Folienschlauchbeutel 120 ein Adapter 122 aufgesetzt, an den der Schlauch 110 angeschlossen ist.

Für eine effiziente Behandlung einer Wunde, Dermatose oder dergleichen einer in dem Folienschlauchbeutel 120 aufgenommenen Extremität muss ein dynamischer Stoffaustausch gewährleistet werden, bei dem die zugeführten und abreagierten reaktiven Gase fortlaufend durch frisch zugeführte reaktive Gase ersetzt werden. Dazu weist der Folienschlauchbeutel 120 eine zum Ableiten der ggf. bereits abreagierten reaktiven Gase vom Behandlungsort ausgelegte Auslassöffnung 130 auf, die von einem als Neutralisierungseinrichtung dienenden Aktivkohlefilter 140 abgedeckt ist.

Der Folienschlauchbeutel 120 kann aus einer wasserdampfdurchlässigen Folie bestehen, wie sie beispielsweise in der EP 12 002 332.0 beschrieben ist.

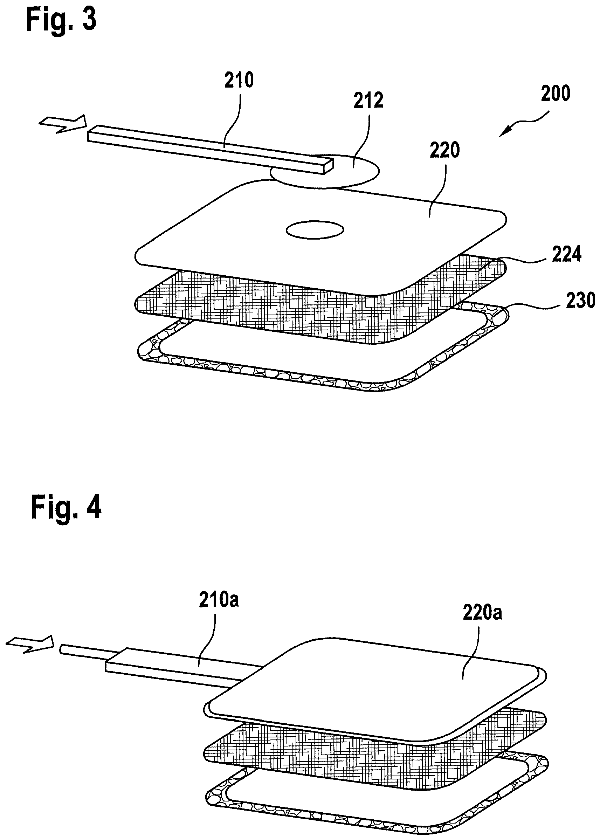

In Fig. 3 ist eine erfindungsgemäße Behandlungsvorrichtung 200 in Form eines zur lokalen, nach außen begrenzten Behandlung von Wunden mit reaktiven Gasen in einem relativ dichten System ausgelegten Patchs dargestellt. Das Reaktionsgas wird von einer Plasmaquelle über einen Schlauch 210, einen Adapter 212 und eine Verteilzone 224 der zu behandelnden Wunde zugeführt. Der Adapter 212 ist gasdicht auf eine okklusive Folie 220 aufgesetzt, wobei die okklusive Folie 220 im Bereich des Adapters 212 eine Einlassöffnung aufweist, durch die das reaktive Gas durch die Verteilfolie 224 zu der zu behandelnden Wunde gelangen kann. Die Verteilzone 224 kann entsprechend dem Stand der Technik gemäß DE 10 2009 019 646 ausgeführt sein.

An der der Wunde zugewandten Begrenzungsfläche der Verteilfolie 224 ist ein den Rand der Folie 220 umlaufender offenporiger Aktivkohleschaum angeordnet, über den die Folie 220 an der die Wunde umlaufenden Haut fixierbar ist. Durch den offenporigen Aktivkohleschaum können die über den Schlauch 210 und die Folie 220 zugeführten reaktiven Gase den Behandlungsbereich wieder verlassen und unter dem Einfluss der Aktivkohle neutralisiert werden, so dass Ärzte, Schwestern und Pfleger keinen Belastungen durch die reaktiven Gase ausgesetzt werden.

Die in Fig. 4 dargestellte Ausführungsform der Erfindung unterscheidet sich im Wesentlichen dadurch von der anhand der Fig. 3 erläuterten Ausführungsform, dass anstelle der okklusiven Folie 220 ein Wundverband 220a eingesetzt ist, der einen Schaum, einen Superabsorber, ein Vlies oder dergleichen enthalten kann. Die reaktiven Gase werden bei der in Fig. 4 dargestellten Ausführungsform der Erfindung über einen Folienschlauch 210a zugeführt.

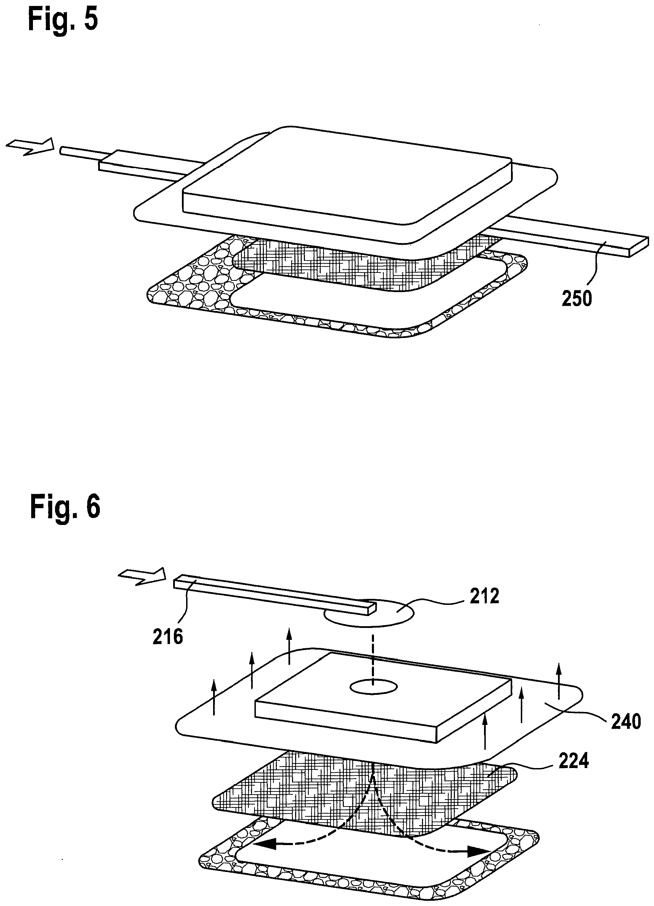

Die in Fig. 5 dargestellte Ausführungsform der Erfindung unterscheidet sich dadurch von der anhand der Fig. 4 erläuterten Ausführungsform, dass zusätzlich zu dem offenporigen Aktivkohleschaum ein flexibler Auslassschlauch für die reaktiven Gase vorgesehen ist, in dem ein Aktivkohlefilter integriert sein kann, um auch dort die reaktiven Gase zu neutralisieren.

Die in Fig. 6 dargestellte Ausführungsform der Erfindung umfasst zusätzlich zu den anhand der Fig. 4 erläuterten Bestandteilen eine als Haftfolie 240 ausgeführte Haftschicht. Die Haftschicht bildet die dem Behandlungsbereich abgewandte äußere Begrenzungsfläche der Abdeckeinrichtung. Sie ist aus einem gasdurchlässigen Material ausgeführt. Mit der Haftschicht wird mit Aktivkohle versetzter Schaum abgedeckt. Bei dieser Ausführungsform reicht die Haftschicht über den aktivkohlehaltigen Schaumrand hinaus und umläuft diesen Schaumrand auf der dem Behandlungsbereich abgewandten Seite. Auf der der äußeren Begrenzungsfläche hautseitigen Begrenzungsfläche ist die Haftschicht haftend ausgeführt und kann an der den aktivkohlehaltigen Schaum umlaufenden Haut befestigt werden, um so die gesamte Abdeckeinrichtung 70 an der den Behandlungsbereich umlaufenden Haut zu fixieren. Bei der in Fig. 6 dargestellten Ausführungsform der Erfindung wird das Reaktionsgas ähnlich wie bei der anhand der Fig. 3 dargestellten Ausführungsform von einer Plasmaquelle über einen Schlauch 210, einen Adapter 212 und eine Verteilzone 224 der zu behandelnden Wunde zugeführt.

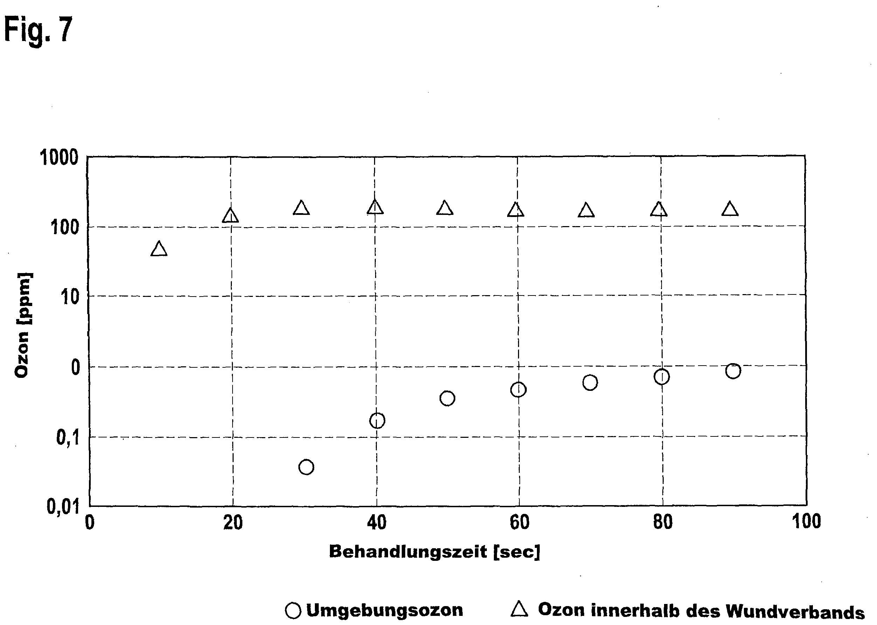

Fig. 7 zeigt den Ozonkonzentrationsverlauf in einer Ausführungsform wie in Fig. 3 dargestellt. In dieser Ausführungsform ist die Neutralisierungseinrichtung als ein mit Aktivkohle versetzter Schaumrand von 1,25 cm ausgeführt und so im Wundverband, welcher über der Wunde fixiert wird, integriert. Die reaktiven Spezies wie z.B. Ozon werden durch den offenporigen Schaum gefiltert und neutralisiert. Dabei steigt die Ozonkonzentration über der Wundoberfläche auf rund 200 ppm während sich die Umgebungsozonkonzentration in einem definierten Volumen von 36 dm³ ohne Luftzirkulation mit einer Steigung von 0,008 ppm zunimmt. Die Neutralisierungseinrichtung hat in dieser Ausführungsform bei einer Wirkfläche von 0,6 cm³ einen Wirkungsgrad von 99,75 %.

Für eine gleichmäßige Behandlung über eine größere Fläche kann die Applikationseinrichtung eine zum Verteilen der reaktiven Gase über eine vorgegebene Fläche ausgelegte Verteilschicht aufweisen. Diese Verteilschicht kann beispielsweise entsprechend der DE 10 2009 019 646 ausgeführt sein. Bei den vorstehend beschriebenen Ausführungsformen der Erfindung, die für eine indirekte Plasmabehandlung ausgelegt sind, ist der Reaktionsgasgenerator zweckmäßigerweise über einen Schlauch mit einer Einlassöffnung der Applikationseinrichtung verbunden. Die Auslassöffnung kann auch in Form eines zusätzlichen Schlauchlumens in dem Schlauch, in einem Anschlussstück für den Schlauch und/oder in dem Fixierrand integriert sein. Wenn die Applikationseinrichtung in Form eines Patches oder Wundverbands ausgeführt ist, kann die Abdeckeinrichtung eine okklusive Folie aufweisen, welche im Bereich eines den Behandlungsbereich umlaufenden Fixierrands an der Haut fixierbar ist. Die Fixierung kann auch über einen offenporigen und mit Aktivkohle versetzten, eine Neutralisierungseinrichtung bildenden Schaumrand erfolgen. In diesem Fall bildet der Schaumrand eine Vielzahl von Auslassöffnungen.

Bei Ausführung der Applikationseinrichtung als Wundverband, kann die Abdeckeinrichtung zusätzlich zur okklusiven Folie einen Schaum, einen Superabsorber und/oder ein Vlies aufweisen. Bei stark exsudierenden Wunden muss ein Rückfluss der Exsudate in den zur Zuführung der reaktiven Gase verwendeten Schlauch verhindert werden. Der Schlauch muss steril verschlossen sein, um eine sichere nächste Behandlung zu gewährleisten. Wunden in der Granulationsphase sondern eine große Menge an Exsudat, bestehend primär aus abgestorbenen Zellen, Bakterien und Protein, ab. Für die Einlass- und Auslassöffnungen des reaktiven Gases sind Vorkehrungen zu treffen, um das Eindringen des Exsudats und anderer Flüssigkeiten zu verhindern. Eine solche Vorkehrung kann die Verwendung eines Klappenventils oder unterschiedlicher Oberflächenbeschichtungen sein.

Falls das Patch, welches zur Zuführung reaktiver Gase ausgelegt ist, längere Zeit auf der Wunde bleibt, kann der zur Zuführung der reaktiven Gase eingesetzte Schlauch aus flexibleren Materialien hergestellt sein, beispielsweise einen Folienschlauch umfassen. Das instabile Material verhindert Druckstellen und erhöht den Tragekomfort für den Patienten. Sofern eine erfindungsgemäße Vorrichtung eine als Patch ausgeführte Anordnung aufweist, kann die Abdeckeinrichtung eine die dem Behandlungsbereich abgewandte äußere Begrenzungsfläche davon bildende, vorzugsweise gasdurchlässige, insbesondere folienartige Haftschicht aufweisen, welche den ggf. einen den Behandlungsbereich umlaufenden, mit Aktivkohle versetzten offenporigen Schaum abdeckenden und auf der dem Behandlungsbereich abgewandten Seite umlaufenden Fixierrand bildet.

Die adäquate Behandlung chronischer Wunden verursacht durch venöse oder arterielle Probleme, wie zum Beispiel der venöse, arterielle oder gemischte Ulcus cruris, umfasst neben der Plasmatherapie in vielen Fällen auch zusätzlich eine Kompressionstherapie. Zur Behandlung mit reaktiven Gasen unter dem Kompressionsverband ohne Druckschmerzen wird in diesem Fall das Gas über einen dünnen Schlauch oder einen Folienschlauch eingeleitet. Der Schlauch ist flexibel und verhindert Druckschmerzen. Der Folienschlauch kann mit Hilfe einer Stütze, zum Beispiel einem Schaum, formstabiler ausgeführt sein. Um den dynamischen Stoffaustausch an der Grenzschicht sicherzustellen, müssen auch die Ausführungsvarianten erfindungsgemäßer Vorrichtungen mit einem Wundverband, vor allem mit selbstklebendem Rand oder wenn Kompressionsverbände angelegt werden, über eine oder mehrere Auslassöffnungen verfügen. Dazu kann die Auslassöffnung ähnlich wie die Einlassöffnung über einen dünnen flexiblen Schlauch oder ähnliches verfügen. In diesem Fall kann die Neutralisierungseinrichtung direkt in den Auslassschlauch integriert sein.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- Fig. 1: eine Behandlungsvorrichtung gemäß einer ersten Ausführungsform der Erfindung,
- Fig. 2: eine Behandlungsvorrichtung gemäß einer zweiten Ausführungsform der Erfindung,
- Fig. 3: eine Behandlungsvorrichtung gemäß einer dritten Ausführungsform der Erfindung,
- Fig. 4: eine weitere Behandlungsvorrichtung gemäß einer vierten Ausführungsform der Erfindung,
- Fig. 5: eine Behandlungsvorrichtung gemäß einer fünften Ausführungsform der Erfindung,
- Fig. 6: eine Behandlungsvorrichtung gemäß einer sechsten Ausführungsform der Erfindung, und
- Fig. 7: den Wirkungsgrad der Neutralisierungseinrichtung in einer Ausführungsform.

Die in Fig. 1 dargestellte Behandlungsvorrichtung umfasst eine Düseneinrichtung 12, der reaktive Gase aus einem in Form einer Plasmaquelle ausgeführten Reaktionsgasgenerator zugeführt werden, wie durch den Pfeil A angedeutet. Mit der Düseneinrichtung 12 werden die reaktiven Gase in Form eines offenen Gasstrahls zur Wundoberfläche gefördert. Auf die Düseneinrichtung 12 ist eine trichterförmige Gasführungsvorrichtung aufgesteckt. Die Düseneinrichtung 12 und Gasführungsvorrichtung 16 bilden zusammen eine Applikationseinrichtung im Sinne der Erfindung.

Die Gasführungseinrichtung umfasst einen sich in Richtung auf die zu behandelnde Wunde erweiternden Kunststofftrichter 15, der an seiner äußeren Begrenzungsfläche von einem mit Aktivkohle versehenen offenporigen Schaum 14 abgedeckt ist, wobei sich der offenporige Schlauch 14 über den Rand des Kunststofftrichters 15 hinaus in Richtung auf die Wunde erstreckt. Mit Hilfe des mit Aktivkohle versetzten offenporigen Schaums werden die den Behandlungsbereich verlassenden reaktiven Gase neutralisiert, d. h. zu stabilen Stoffen umgesetzt. Beispielsweise wird Ozon mit Aktivkohle zu Sauerstoff und Kohlendioxid umgesetzt.

Bei der in Fig. 2 dargestellten Ausführungsform der Erfindung werden die in einem Reaktionsgasgenerator in Form einer Plasmaquelle hergestellten reaktiven Gase, wie durch den Pfeil A angedeutet, über einen Schlauch 110 in einen Folienschlauchbeutel 120 eingeleitet. Dabei ist auf den Folienschlauchbeutel 120 ein Adapter 122 aufgesetzt, an den der Schlauch 110 angeschlossen ist.

Für eine effiziente Behandlung einer Wunde, Dermatose oder dergleichen einer in dem Folienschlauchbeutel 120 aufgenommenen Extremität muss ein dynamischer Stoffaustausch gewährleistet werden, bei dem die zugeführten und abreagierten reaktiven Gase fortlaufend durch frisch zugeführte reaktive Gase ersetzt werden. Dazu weist der Folienschlauchbeutel 120 eine zum Ableiten der ggf. bereits abreagierten reaktiven Gase vom Behandlungsort ausgelegte Auslassöffnung 130 auf, die von einem als Neutralisierungseinrichtung dienenden Aktivkohlefilter 140 abgedeckt ist.

Der Folienschlauchbeutel 120 kann aus einer wasserdampfdurchlässigen Folie bestehen, wie sie beispielsweise in der EP 12 002 332.0 beschrieben ist.

In Fig. 3 ist eine erfindungsgemäße Behandlungsvorrichtung 200 in Form eines zur lokalen, nach außen begrenzten Behandlung von Wunden mit reaktiven Gasen in einem relativ dichten System ausgelegten Patchs dargestellt. Das Reaktionsgas wird von einer Plasmaquelle über einen Schlauch 210, einen Adapter 212 und eine Verteilzone 224 der zu behandelnden Wunde zugeführt. Der Adapter 212 ist gasdicht auf eine okklusive Folie 220 aufgesetzt, wobei die okklusive Folie 220 im Bereich des Adapters 212 eine Einlassöffnung aufweist, durch die das reaktive Gas durch die Verteilfolie 224 zu der zu behandelnden Wunde gelangen kann. Die Verteilzone 224 kann entsprechend dem Stand der Technik gemäß DE 10 2009 019 646 ausgeführt sein.

An der der Wunde zugewandten Begrenzungsfläche der Verteilfolie 224 ist ein den Rand der Folie 220 umlaufender offenporiger Aktivkohleschaum angeordnet, über den die Folie 220 an der die Wunde umlaufenden Haut fixierbar ist. Durch den offenporigen Aktivkohleschaum können die über den Schlauch 210 und die Folie 220 zugeführten reaktiven Gase den Behandlungsbereich wieder verlassen und unter dem Einfluss der Aktivkohle neutralisiert werden, so dass Ärzte, Schwestern und Pfleger keinen Belastungen durch die reaktiven Gase ausgesetzt werden.

Die in Fig. 4 dargestellte Ausführungsform der Erfindung unterscheidet sich im Wesentlichen dadurch von der anhand der Fig. 3 erläuterten Ausführungsform, dass anstelle der okklusiven Folie 220 ein Wundverband 220a eingesetzt ist, der einen Schaum, einen Superabsorber, ein Vlies oder dergleichen enthalten kann. Die reaktiven Gase werden bei der in Fig. 4 dargestellten Ausführungsform der Erfindung über einen Folienschlauch 210a zugeführt.

Die in Fig. 5 dargestellte Ausführungsform der Erfindung unterscheidet sich dadurch von der anhand der Fig. 4 erläuterten Ausführungsform, dass zusätzlich zu dem offenporigen Aktivkohleschaum ein flexibler Auslassschlauch für die reaktiven Gase vorgesehen ist, in dem ein Aktivkohlefilter integriert sein kann, um auch dort die reaktiven Gase zu neutralisieren.

Die in Fig. 6 dargestellte Ausführungsform der Erfindung umfasst zusätzlich zu den anhand der Fig. 4 erläuterten Bestandteilen eine als Haftfolie 240 ausgeführte Haftschicht. Die Haftschicht bildet die dem Behandlungsbereich abgewandte äußere Begrenzungsfläche der Abdeckeinrichtung. Sie ist aus einem gasdurchlässigen Material ausgeführt. Mit der Haftschicht wird mit Aktivkohle versetzter Schaum abgedeckt. Bei dieser Ausführungsform reicht die Haftschicht über den aktivkohlehaltigen Schaumrand hinaus und umläuft diesen Schaumrand auf der dem Behandlungsbereich abgewandten Seite. Auf der der äußeren Begrenzungsfläche hautseitigen Begrenzungsfläche ist die Haftschicht haftend ausgeführt und kann an der den aktivkohlehaltigen Schaum umlaufenden Haut befestigt werden, um so die gesamte Abdeckeinrichtung 70 an der den Behandlungsbereich umlaufenden Haut zu fixieren. Bei der in Fig. 6 dargestellten Ausführungsform der Erfindung wird das Reaktionsgas ähnlich wie bei der anhand der Fig. 3 dargestellten Ausführungsform von einer Plasmaquelle über einen Schlauch 210, einen Adapter 212 und eine Verteilzone 224 der zu behandelnden Wunde zugeführt.

Fig. 7 zeigt den Ozonkonzentrationsverlauf in einer Ausführungsform wie in Fig. 3 dargestellt. In dieser Ausführungsform ist die Neutralisierungseinrichtung als ein mit Aktivkohle versetzter Schaumrand von 1,25 cm ausgeführt und so im Wundverband, welcher über der Wunde fixiert wird, integriert. Die reaktiven Spezies wie z.B. Ozon werden durch den offenporigen Schaum gefiltert und neutralisiert. Dabei steigt die Ozonkonzentration über der Wundoberfläche auf rund 200 ppm während sich die Umgebungsozonkonzentration in einem definierten Volumen von 36 dm³ ohne Luftzirkulation mit einer Steigung von 0,008 ppm zunimmt. Die Neutralisierungseinrichtung hat in dieser Ausführungsform bei einer Wirkfläche von 0,6 cm³ einen Wirkungsgrad von 99,75 %.

Die Erfindung ist nicht auf die anhand der Zeichnung erläuterten Ausführungsformen beschränkt. Vielmehr ist auch an den Einsatz erfindungsgemäßer Vorrichtungen zur direkten Plasmabehandlung gedacht. Es können andersartige reaktive Gase eingesetzt werden. Die Applikationseinrichtung kann beliebig an die Form der Wunden und die anatomischen Verhältnisse angepasst werden.

### Bezugszeichenliste

- 12: Düseneinrichtung
- 15: Gasführungsvorrichtung
- 110: Schlauch
- 120: Folienschlauchbeutel
- 122: Adapter
- 130: Auslassöffnung
- 140: Aktivkohlefilter
- 200: Behandlungsvorrichtung
- 210: Schlauch
- 210a: Folienschlauch
- 212: Adapter
- 220: Folie
- 220a: Wundverband
- 224: Verteilfolie

## Patentansprüche

1. Vorrichtung zur human- und/oder tiermedizinischen Behandlung, insbesondere zur Behandlung von Wunden und/oder Dermatosen, mit einem zum Erzeugen reaktiver Gase ausgelegten Reaktionsgasgenerator und einer Applikationseinrichtung zum Applizieren der reaktiven Gase in einem Behandlungsbereich, mit einer zum Neutralisieren reaktiver Gase ausgelegte Neutralisierungseinrichtung, mit der die Konzentration reaktiver Gase außerhalb des Behandlungsbereichs reduzierbar ist, **dadurch gekennzeichnet, dass** die Applikationseinrichtung eine Abdeckeinrichtung, wie etwa eine Abdeckfolie und/oder einen Wundverband zum Abdecken des Behandlungsorts aufweist, die Abdeckeinrichtung mindestens eine zum Einleiten der reaktiven Gase zum Behandlungsbereich ausgelegte Einlassöffnung und mindestens eine zum Ableiten der reaktiven Gase aus dem Behandlungsbereich ausgelegte Auslassöffnung aufweist und der Reaktionsgasgenerator über einen Schlauch mit einer Einlassöffnung der Applikationseinrichtung verbunden ist.

2. Vorrichtung nach Anspruch 1, **dadurch gekennzeichnet, dass** der Reaktionsgasgenerator eine Plasmaquelle, ggf. in Form eines piezoelektrischen Transformators zur Erzeugung eines Atmosphärendruck Plasmas, aufweist.

3. Vorrichtung nach Anspruch 1 oder 2, **dadurch gekennzeichnet, dass** die Neutralisierungseinrichtung Aktivkohle aufweist.

4. Vorrichtung nach einem der vorhergehenden Ansprüche, **dadurch gekennzeichnet, dass** die Applikationseinrichtung eine zum Erzeugen des auf den Behandlungsort gerichteten Strahls der reaktiven Gase ausgelegte Düseneinrichtung aufweist.

5. Vorrichtung nach Anspruch 4, **dadurch gekennzeichnet, dass** die Applikationseinrichtung einen den Reaktionsgasgenerator mit der Düseneinrichtung verbindenden Schlauch aufweist.

6. Vorrichtung nach Anspruch 4 oder 5, **dadurch gekennzeichnet, dass** die Neutralisierungseinrichtung ein den Strahl reaktiver Gase umlaufendes, vorzugsweise sich trichterförmig in Richtung auf den Behandlungsort erweiterndes Gehäuse aufweist.

7. Vorrichtung nach Anspruch 6, **dadurch gekennzeichnet, dass** das Gehäuse einen mit Aktivkohle versetzten Schaum aufweist.

8. Vorrichtung nach Anspruch 6 oder 7, **dadurch gekennzeichnet, dass** das Gehäuse einen Kunststofftrichter aufweist, dessen äußere Begrenzungsfläche zumindest teilweise von dem mit Aktivkohle versetzten offenporigen Schaum abgedeckt ist.

9. Vorrichtung nach Anspruch 8, **dadurch gekennzeichnet, dass** der Kunststofftrichter von mindestens einer von dem mit Aktivkohle versetzten Schaum abgedeckten Öffnung durchsetzt ist.

10. Vorrichtung nach Anspruch 8 oder 9, **dadurch gekennzeichnet, dass** der Kunststofftrichter auf die Düseneinrichtung lösbar aufsteckbar ist.

11. Vorrichtung nach einem der vorhergehenden Ansprüche, **dadurch gekennzeichnet, dass** die Applikationseinrichtung eine zur Beeinflussung der Strömung der reaktiven Gase ausgelegte Strömungsleiteinrichtung aufweist.

12. Vorrichtung nach einem der vorhergehenden Ansprüche, **dadurch gekennzeichnet, dass** die Neutralisierungseinrichtung im Bereich der Auslassöffnung angeordnet ist, und diese vorzugsweise abdeckt.

13. Vorrichtung nach einem der vorhergehenden Ansprüche, **dadurch gekennzeichnet, dass** die Abdeckeinrichtung einen vorzugsweise wasserdampfdurchlässigen Folienschlauch aufweist, der vorzugsweise an einem Ende verschlossen ist.

14. Vorrichtung nach einem der vorhergehenden Ansprüche, **dadurch gekennzeichnete, dass** die Abdeckeinrichtung nach Art eines Extremitätenbeutels, einer Socke, eines Handschuhs, einer Weste oder einer Hose ausgeführt ist.

15. Vorrichtung nach einem der vorhergehenden Ansprüche, **dadurch gekennzeichnet, dass** die Abdeckeinrichtung im Bereich eines Fixierrands an der Haut fixierbar ist, und vorzugsweise ein Verschlussband und/oder ein Haftmittel, wie etwa eine selbstklebenden Folie, aufweist.

16. Vorrichtung nach einem der vorhergehenden Ansprüche, **dadurch gekennzeichnet, dass** die Applikationseinrichtung eine zum Verteilen der reaktiven Gase über eine vorgegebene Fläche ausgelegte Verteilschicht aufweist.

17. Vorrichtung nach einem der vorhergehenden Ansprüche, **dadurch gekennzeichnet, dass** mindestens eine Auslassöffnung, ggf. in Form eines zusätzlichen Schlauchlumens in dem Schlauch, in ein Anschlussstück für den Schlauch und/oder in den Fixierrand integriert ist.

18. Vorrichtung nach einem der vorhergehenden Ansprüche, **dadurch gekennzeichnet, dass** die Abdeckeinrichtung im Bereich eines den Behandlungsbereich umlaufenden Fixierrands an der Haut fixierbar ist.

19. Vorrichtung nach einem der vorhergehenden Ansprüche, **dadurch gekennzeichnet, dass** die Abdeckeinrichtung einen Schaum, einen Superabsorber und/oder ein Vlies aufweist.

20. Vorrichtung nach Anspruch 18 oder 19, **dadurch gekennzeichnet, dass** der Abdeckeinrichtung eine Rückflusssperre zugeordnet ist, mit der dem Auslaufen von Wundexsudat aus der Auslassöffnung entgegengewirkt wird, wie etwa ein Klappenventil.

21. Vorrichtung nach Anspruch 18 bis 20, **dadurch gekennzeichnet, dass** der Fixierrand einen mit Aktivkohle versetzten offenporigen Schaum aufweist.

22. Vorrichtung nach einem der Ansprüche 18 bis 21, **dadurch gekennzeichnet, dass** die Abdeckeinrichtung eine die dem Behandlungsbereich abgewandte äußere Begrenzungsfläche davon bildende, vorzugsweise gasdurchlässige, insbesondere folienartige Haftschicht aufweist, welche den ggf. einen den Behandlungsbereich umlaufenden mit Aktivkohle versetzten offenporigen Schaum abdeckenden und umlaufenden Fixierrand bildet.

23. Behandlungskit zur Herstellung einer Vorrichtung nach einem der vorhergehenden Ansprüche mit einer Applikationseinrichtung und einer Neutralisierungseinrichtung.

## Claims

1. A device for the medical treatment of humans and/or animals, in particular for the treatment of wounds and/or dermatoses, comprising a reaction gas generator configured for generating reactive gases, and an application device for applying said reactive gases in a treatment area, and comprising a neutralisation device configured for neutralising reactive gases and by means of which the concentration of reactive gases outside the treatment area can be reduced, **characterised in that** the application device comprises a covering device, such as a covering film and/or a wound dressing for covering the treatment site, said covering device comprises at least one inlet port configured for introducing the reactive gases into the treatment area and at least one outlet port configured for removing the reactive gases from the treatment area, and the reaction gas generator is connected to an inlet port of the application device via a tube.

2. The device according to claim 1, **characterised in that** the reaction gas generator comprises a plasma source, possibly in the form of a piezoelectric transformer for generating an atmospheric pressure plasma.

3. The device according to claim 1 or 2, **characterised in that** the neutralisation device comprises activated charcoal.

4. The device according to one of the preceding claims, **characterised in that** the application device comprises a nozzle device configured for generating the jet of reactive gases that is directed towards the treatment site.

5. The device according to claim 4, **characterised in that** the application device comprises a tube connecting the reaction gas generator to the nozzle device.

6. The device according to claim 4 or 5, **characterised in that** the neutralisation device comprises a housing which surrounds the jet of reactive gases and preferably widens in a funnel-like manner in the direction of the treatment site.

7. The device according to claim 6, **characterised in that** the housing comprises a foam charged with activated charcoal.

8. The device according to claim 6 or 7, **characterised in that** the housing comprises a plastic funnel whose outer surface is at least partially covered by the open-cell foam that is charged with activated charcoal.

9. The device according to claim 8, **characterised in that** the plastic funnel is penetrated by at least one port covered by the foam charged with activated charcoal.

10. The device according to claim 8 or 9, **characterised in that** the plastic funnel can be detachably mounted onto the nozzle device.

11. The device according to one of the preceding claims, **characterised in that** the application device comprises a flow control device configured for influencing the flow of reactive gases.

12. The device according to one of the preceding claims, **characterised in that** the neutralisation device is arranged in the area of the outlet port, and preferably covers it.

13. The device according to one of the preceding claims, **characterised in that** the covering device comprises a preferably water vapour-permeable tubular film, one of the ends of which is preferably sealed.

14. The device according to one of the preceding claims, **characterised in that** the covering device is configured as an extremity bag, sock, glove, vest or pair of trousers.

15. The device according to one of the preceding claims, **characterised in that** the covering device can be fastened to the skin in the area of a fastening edge and preferably comprises a closure strip and/or an adhesive, such as a self-adhesive film.

16. The device according to one of the preceding claims, **characterised in that** the application device comprises a distribution layer configured for distributing the reactive gases over a specified area.

17. The device according to one of the preceding claims, **characterised in that** at least one outlet port, possibly in the form of an additional lumen in the tube, is integrated in a connector for the tube and/or in the fastening edge.

18. The device according to one of the preceding claims, **characterised in that** the covering device can be fastened to the skin in the area of a fastening edge surrounding the treatment area.

19. The device according to one of the preceding claims, **characterised in that** the covering device comprises a foam, a superabsorber and/or a non-woven material.

20. The device according to claim 18 or 19, **characterised in that** the covering device is associated with a check valve, such as a flap valve, preventing the outflow of wound exudate from the outlet port.

21. The device according to claims 18 to 20, **characterised in that** the fastening edge comprises an open-cell foam charged with activated charcoal.

22. The device according to claims 18 to 21, **characterised in that** the covering device comprises a preferably gas-permeable, in particular film-like adhesive layer, which forms its outer surface that faces away from the treatment area, which adhesive layer forms the fastening edge which surrounds and covers a/the open-cell foam charged with activated charcoal that surrounds the treatment area.

23. A treatment kit for creating a device according to one of the previous claims having an application device and a neutralisation device.

## Revendications

1. Dispositif de traitement médical destiné à l'être humain et/ou aux animaux, notamment pour le traitement de plaies et/ou de dermatoses, comportant un générateur de gaz réactionnels conçu pour générer des gaz réactifs et un dispositif d'application destiné à l'application desdits gaz réactifs dans une zone de traitement, et comportant un dispositif de neutralisation conçu pour neutraliser des gaz réactifs et permettant de réduire la concentration en gaz réactifs en dehors de la zone de traitement ; **caractérisé en ce que** le dispositif d'application présente un dispositif de recouvrement, tel qu'un film de recouvrement et/ou un pansement pour recouvrir le site de traitement, le dispositif de recouvrement présente au moins une ouverture d'entrée conçue pour l'introduction des gaz réactifs dans la zone de traitement et au moins une ouverture de sortie conçue pour l'évacuation des gaz réactifs de la zone de traitement, et le générateur de gaz réactionnels est relié à une ouverture d'entrée du dispositif d'application par le biais d'un tuyau.

2. Dispositif selon la revendication 1, **caractérisé en ce que** le générateur de gaz réactionnels présente une source de plasma, éventuellement sous la forme d'un transformateur piézoélectrique destiné à générer un plasma à pression atmosphérique.

3. Dispositif selon la revendication 1 ou 2, **caractérisé en ce que** le dispositif de neutralisation présente du charbon actif.

4. Dispositif selon l'une des revendications précédentes, **caractérisé en ce que** le dispositif d'application présente un dispositif formant buse conçu pour générer le jet de gaz réactifs dirigé vers le site de traitement.

5. Dispositif selon la revendication 4, **caractérisé en ce que** le dispositif d'application présente un tuyau reliant le générateur de gaz réactionnels au dispositif formant buse.

6. Dispositif selon la revendication 4 ou 5, **caractérisé en ce que** le dispositif de neutralisation présente un boîtier qui entoure le jet de gaz réactifs en s'évasant de préférence à la manière d'un entonnoir en direction du site de traitement.

7. Dispositif selon la revendication 6, **caractérisé en ce que** le boîtier présente une mousse chargée de charbon actif.

8. Dispositif selon la revendication 6 ou 7, **caractérisé en ce que** le boîtier présente un entonnoir en matière plastique dont la surface extérieure est au moins partiellement recouverte par la mousse à pores ouverts chargée de charbon actif.

9. Dispositif selon la revendication 8, **caractérisé en ce que** l'entonnoir en plastique est traversé par au moins une ouverture recouverte par la mousse chargée de charbon actif.

10. Dispositif selon la revendication 8 ou 9, **caractérisé en ce que** l'entonnoir en matière plastique peut être emboîté de manière amovible sur le dispositif formant buse.

11. Dispositif selon l'une des revendications précédentes, **caractérisé en ce que** le dispositif d'application présente un dispositif de guidage d'écoulement conçu pour agir sur l'écoulement des gaz réactifs.

12. Dispositif selon l'une des revendications précédentes, **caractérisé en ce que** le dispositif de neutralisation est disposé au niveau de l'ouverture de sortie, de préférence en la recouvrant.

13. Dispositif selon l'une des revendications précédentes, **caractérisé en ce que** le dispositif de recouvrement présente un film tubulaire de préférence perméable à la vapeur d'eau, qui est de préférence fermé à une extrémité.

14. Dispositif selon l'une des revendications précédentes, **caractérisé en ce que** le dispositif de recouvrement est conçu à la manière d'un sachet pour extrémité, d'une chaussette, d'un gant, d'un gilet ou d'un pantalon.

15. Dispositif selon l'une des revendications précédentes, **caractérisé en ce que** le dispositif de recouvrement peut être fixé à la peau au niveau d'un bord de fixation, et présente de préférence un ruban de fermeture et/ou un adhésif, tel qu'un film autocollant.

16. Dispositif selon l'une des revendications précédentes, **caractérisé en ce que** le dispositif d'application présente une couche de répartition conçue pour répartir les gaz réactifs sur une superficie prédéterminée.

17. Dispositif selon l'une des revendications précédentes, **caractérisé en ce qu'**au moins une ouverture de sortie, éventuellement sous la forme d'une lumière supplémentaire présente dans le tuyau, est intégrée dans un embout de raccordement pour le tuyau et/ou dans le bord de fixation.

18. Dispositif selon l'une des revendications précédentes, **caractérisé en ce que** le dispositif de recouvrement peut être fixé sur la peau au niveau d'un bord de fixation entourant la zone de traitement.

19. Dispositif selon l'une des revendications précédentes, **caractérisé en ce que** le dispositif de recouvrement présente une mousse, un superabsorbant et/ou un non-tissé.

20. Dispositif selon la revendication 18 ou 19, **caractérisé en ce que** le dispositif de recouvrement est doté d'un dispositif anti-retour empêchant toute fuite d'exsudat issu de la plaie par l'ouverture de sortie, tel qu'une soupape à clapet.

21. Dispositif selon les revendications 18 à 20, **caractérisé en ce que** le bord de fixation présente une mousse à pores ouverts chargée de charbon actif.

22. Dispositif selon l'une des revendications 18 à 21, **caractérisé en ce que** le dispositif de recouvrement présente une couche adhésive de préférence perméable aux gaz, notamment sous la forme d'un film, qui forme la surface extérieure dudit dispositif qui est détournée de la zone de traitement, ladite couche adhésive formant le bord de fixation qui entoure et recouvre une/ladite mousse à pores ouverts chargée de charbon actif entourant la zone de traitement.

23. Trousse de traitement permettant de réaliser un dispositif selon l'une des revendications précédentes comportant un dispositif d'application et un dispositif de neutralisation.
